# EUROPÄISCHE PATENTANMELDUNG

(11) **EP 1 875 908 A1**
(43) Veröffentlichungstag der Anmeldung: **09.01.2008**
(21) Anmeldenummer: 06450093.7
(22) Anmeldetag: 05.07.2006
(51) Int. Cl.: A61K 31/352, A61P 29/00, A61P 3/00

(54) **Verwendung von Chrysin**

(71) Anmelder: Huber, Johannes, 1130 Wien (AT); Valora Unternehmensberatung und -beteiligung AG, 1040 Wien (AT)
(72) Erfinder: Huber, Johannes, 1130 Wien (AT)
(74) Vertreter: Sonn & Partner Patentanwälte

(57) **Zusammenfassung**

Beschrieben wird die Verwendung von Chrysin-Präparaten zur Herstellung eines Medikaments zur Behandlung oder Verhinderung von Mastalgie.

## Beschreibung

Die Erfindung betrifft die Behandlung oder Verhinderung von Mastalgie.

Mit Mastalgie (Mastodynie oder Mammalgie) werden Schmerzen in den Brüsten bezeichnet, die mit Spannungs- und Schwellungsgefühlen zusammen hängen. Die Schmerzen werden häufiger prämenstruell als kontinuierlich empfunden, im Allgemeinen unterscheidet man zwei klinische Muster: Zyklisch und Nicht-Zyklisch.

Beim zyklischen Muster der Mastalgie ist der Schmerz stets am schlimmsten vor jedem Menstruationszyklus. Dies kann in Zusammenhang mit dem natürlichen weiblichen Menstruationszyklus stehen oder wird durch Hormontabletten ausgelöst. Die nichtzyklische Form ist unabhängig von Menstruationszyklen und kann unter anderem durch den unterliegenden Muskel Trauma und daraus resultierenden Hämatomen oder durch Mastitis verursacht sein.

Die Ätiologie der Mastalgie wird unterschiedlich diskutiert: einerseits werden Wassereinlagerungen, Ödembildung und Dilatation der venösen Gefäße dafür verantwortlich gemacht, andererseits wird der durch die Sexualsteroide ausgelöste Proliferationsdruck als Ursache genannt, der zu einer Zunahme des interstitiellen Gewebes und damit auch zu mastopathischen Veränderungen führt.

Zu den Therapieoptionen gehören demnach entweder eine vollständige oder eine partielle Reduktion der endogenen Steroidfluktuation (Pye et al., Lanet 2(1985):373-377).

Gestagene werden deswegen als eine therapeutische Möglichkeit herangezogen, weil sie der Östrogenwirkung physiologisch entgegentreten und die Vasodilatation und die Flüssigkeitsansammlung reduzieren bzw. verhindern. Gestagene werden entweder oral-zytisch oder topisch in Form eines für die Behandlung der Mastalgie zugelassenen Präparates (Progestogel) angeboten. Weitere topisch zu verabreichende Stoffe sind in der US 2004/0018991 beschrieben.

Auch die oralen Kombinationspräparate zur Ovualtionssuppression sind eine Therapieoption (Gateley et al., J. R. Soc. Med. 85 (1992) : 12-15).

Die vollständige Suppression der ovarialen Aktivität kann mit GNRH-Analoga erreicht werden, die ebenfalls zur Behandlung mastalgischer Beschwerden andiskutiert wurden (Blichert-Toft et al., Acta. Obstet. Gynecol. Scand. Suppl. 123(1984):185-188).

Allerdings sind die Nebenwirkungen bei dieser Behandlungsform groß.

Prolaktin-Antagonisten werden ebenfalls zur Behandlung der Mastalgie herangezogen (Mansel et al., Br. J. Surg. 65(1978):724-727).

Eine interessante Form zur Therapie der Mastalgie, die sich allerdings nicht durchgesetzt hat, stellt das Danokrain, ein androgen-wirksames synthetisches Präparat, welches auch bei der Behandlung der Endometriose Verwendung findet, seine Anwendung ist durch die mitunter auftretende Gewichtszunahme limitiert. Allerdings handelt es sich dabei bereits um eine androgen wirksame Verbindung.

Die Mastalgie kommt bei 30-40 % der geschlechtsreifen Frauen vor. Der Bedarf ist damit sehr hoch (Roberts, Br. J. Surg. 74(1984):1020-1022; Nichols et al., BMJ281 (1980): 1450-1453).

Die vorliegende Erfindung stellt sich zur Aufgabe, ein wirksames Behandlungsverfahren bzw. wirksame Mittel zur Verhinderung von Mastalgie (Mastodynie, Mammalgie) zur Verfügung zu stellen, welche effizient ist, den Organismus aber nicht übermäßig belastet und auch über einen längeren Zeitraum angewendet werden kann.

Demgemäß betrifft die vorliegende Erfindung die Verwendung von einem Chrysin-Präparat, zur Herstellung eines Medikaments zur Behandlung oder Verhinderung von Mastalgie.

Chrysin(5,7-Dihydroxyflavon) ist ein potentes Bioflavonoid, welches z.B. aus der Passionsfrucht (Passiflora caerulea) gewonnen werden kann. Chrysin wurde beispielsweise schon verwendet, um das Abnehmen (vor allem bei Frauen) zu erleichtern oder um Stressprobleme (z.B. bei Athleten im Training) zu reduzieren.

Überraschenderweise konnte erfindungsgemäß festgestellt werden, dass mit Chrysin-Präparaten Patientinnen, die unter Mastalgien leiden, behandelt werden konnten, so dass diese Mastalgien verschwanden oder besser wurden, was sich auch objektiv in der Reduktion der durch Mammographie feststellbaren Brustdichte zeigte.

Chrysin kommt auch in der pinea silvestris vor, aufmerksam wurde man auf dieses Flavonoid ursprünglich durch seine androgene Partialwirkung. Diese ist allerdings nicht so stark, dass es zu einer hyperandrogenämischen Belastung der Haut, der Haare etc. führen würde.

Die vorliegende Erfindung betrifft daher auch ein Verfahren zur Verhinderung oder Behandlung von Mastalgie, bei welchem eine effiziente Menge an Chrysin an eine Person verabreicht wird, die unter Mastitis leidet oder die ein Risiko hat, Mastitis-Beschwerden zu entwickeln. Demgemäß betrifft die vorliegende Erfindung auch ein Mittel zur Verhinderung oder Behandlung von Mastitis, welches eine effektive Menge an Chrysin enthält.

Die Art und Weise, mit welcher Chrysin-Präparate eingenommen werden, ist nicht kritisch für die vorliegende Erfindung. Am angenehmsten für die Patientinnen hat sich allerdings die orale Verabreichung erwiesen, da sie mit dem wenigsten Aufwand verbunden ist und am wenigsten invasiv ist. Die erfindungsgemäßen Chrysin-Präparate lassen sich auch in Form von Nahrungs- oder Nahrungsergänzungsmittel einnehmen. Es ist natürlich auch möglich, die erfindungsgemäße Verabreichung von Chrysin-Präparaten zur Behandlung oder Verhinderung von Mastalgien mittels Depot-Präparaten vorzunehmen, die gegebenenfalls auch implantiert werden können. Auch das Vorsehen von transdermalen Verabreichungen (z.B. durch Depot-Pflaster) ist natürlich möglich. Eine topische Anwendung ist ebenfalls bevorzugt. Dabei kann das Chrysin-Präparat gleich lokal gezielt aufgetragen werden, z.B. in Form eines Gels, einer Salbe oder einer Emulsion.

Besonders für die orale Verabreichung haben sich Dosiseinheitformen wie Tabletten, Kapseln, Granulate etc., insbesondere als Nahrungsmittel oder Nahrungsergänzungsmittel, als besonders vorteilhaft erwiesen. Derartige Chrysin-Formulierungen sind seit langem bekannt und kommerziell erhältlich.

Chrysin kann erfindungsgemäß auch vorbeugend gegen Mastalgie verwendet werden, insbesondere da es gut verträglich ist und so auch die Einnahme von Chrysin über längere Zeiträume in der Regel völlig unbedenklich ist.

Die zur Behandlung bzw. Verhinderung der Mastalgie jeweils erforderliche Menge an Chrysin-Präparaten sollte jeweils an die Schwere der Mastalgie bzw. die damit verbundenen Schmerzen bzw. Unannehmlichkeiten gebunden werden (bzw. mit der zu erzielenden Linderung der Beschwerden). Mengen zwischen 1 mg und 10 g täglich sind bevorzugt, insbesondere 5 mg bis 5 g, insbesondere 10 mg bis 1 g. Dosiseinheitsformen werden üblicherweise mit 5, 10, 20, 50, 100, 200 oder 500 mg Chrysin/Einheit verabreicht. Diese Chrysin-Präparate können leicht z.B. als Nahrungsergänzungsmittel z.B. 1-4 mal pro Tag zusammen mit dem Essen aufgenommen werden. Bei oraler Verabreichung werden die Dosiseinheiten vorzugsweise in einer Magensaft-resistenten Form zur Verfügung gestellt. Bei der topischen Verabreichung verwendet man vorzugsweise Salben, Emulsionen oder Gele, die Chrysin in einer wirksamen Menge von 0,5 bis 20 Gew.%, vorzugsweise 1 bis 10 Gew.%, insbesondere 2 bis 8 Gew.% (z.B. eine 5%ige Salbe), enthalten.

Vorzugsweise können der erfindungsgemäßen Chrysin-Präparation auch weitere Wirksubstanzen zugesetzt werden, z.B. geeignete weitere Phytohormone, wie Isoflavone (z.B. Formononetin, Daidzein oder Genistein), oder Gallensäuren (inklusive deren Salze), insbesondere Cholsäure. Diese weiteren Wirksubstanzen werden üblicherweise in bereits bewährten Dosierungen zugegeben (Cholsäure z.B. in der Regel von 1 mg bis 10 g, vorzugsweise von 10 mg bis 5 g, insbesondere von 100 mg bis 1 g; in Suspensionen, Emulsionen, Gelen oder Salben, z.B. in einer Menge von 1 bis 5 mg/ml, insbesondere von 10 bis 100 mg/ml.

Gemäß einem weiteren Aspekt betrifft die vorliegende Erfindung auch ein Lebensmittel, dem Chrysin (bzw. eine Chrysin-Präparation) zugesetzt worden ist. Dieses Lebensmittel kann auf vielerlei Art zur Verfügung gestellt werden, z.B. als Fertiggericht, Aufstrich, Riegel, Burger, Jogurt, als Fruchtsaftgetränk, etc.. Die Dosierung kann selbstverständlich von jedem Lebensmittelfachmann einfach eingestellt werden, z.B. auf Basis der zuvor erwähnten Mengen für die Erwirkung eines Mastalgie-protektiven Effekts. Auch beim Lebensmittel hat sich die Kombination mit Cholsäure als überaus vorteilhaft erwiesen. Neben der Prophylaxe und der Therapie von Mastalgie hat sich die erfindungsgemäße Kombination von Chrysin-Präparaten mit Cholsäure als besonders effizient zur Beschleunigung des Fettabbaus im menschlichen Körper erwiesen. Insbesondere weist die Kombination von Chrysin und Cholsäure eine besondere Wirksamkeit in dieser Hinsicht auf, die aufgrund der bekannten Eigenschaften dieser Verbindungen völlig unerwartet war.

Die vorliegende Erfindung wird anhand der nachfolgenden Beispiele, auf die sie selbstverständlich nicht eingeschränkt ist, näher erläutert.

### Beispiel 1

### Klinische Studie zur Wirkung von Chrysin-Präparaten bei der Behandlung von Mastaglie

### Studiendesign

Eine offene, prospektiv durchgeführte Untersuchung wurde in der Ordination zweier niedergelassener Gynäkologen vorgenommen. Insgesamt wurden 58 Patientinnen zwischen dem 23. und dem 45. Lebensjahr in die Beobachtungsstudie involviert. 18 Frauen klagten über zyklusunabhängige Brustschmerzen, die während des ganzen Monates kontinuierlich oder flukturierend auftraten. 40 Patientinnen berichteten über mastalgische Beschwerden in der 2. Zyklushälfte. Als Ausschlusskriterium galten Frauen unter der Pilleneinhame oder unter anderer hormoneller Medikation, Anwendung von Psychopharmaka, Hyperprolaktinämie, einseitige Brustschmerzen (die Mastaglie musste beidseitig auftreten) sowie eine bereits durchgeführte Operation wegen einer gutartigen Brusterkrankung. Keine der Patientinnen hatte in der persönlichen Anamnese eine bösartige Geschwulsterkrankung der Brust.

Als Einschlusskriterium wurde die Feststellung der Patientin gewertet, dass die Brustspannung so stark ist, das sie als störend empfunden wird.

Vor Beginn der Behandlung wurde eine Hormonuntersuchung gemacht, um eine Hypothyreose, eine Hyperprolaktinämie sowie eine Hypergonadotropie auszuschließen. Keine der Patientinnen zeigte in der punktuellen Blutabnahme einen Östradiolwert über 200 ng/ml. Bei 6 Patientinnen zeigte sich eine LH/FSH-Ratio von mehr als 3, bei keiner Patientin lag das Testosteron über 1,2 ng/ml. 49 Patientinnen waren eumenorrhoisch, 9 Patientinnen oligomenorrhoisch. Bei keiner Frau wurde eine Amenorrhoe festgestellt.

Vor Beginn der Untersuchung wurde auch ein Schwangerschaftstest vorgenommen. Keine der Patientinnen war gravid. Bei jeder Patientin war eine palpatorische Untersuchung der Brust vorgenommen worden, bei 43 Patientinnen war vor Beginn der Therapie eine Mammographie gemacht worden.

Den Patientinnen wurde nach ausführliche Aufklärung 20 mg Chrysin verschrieben, die sie zyklusunabhängig beginnend, am Morgen einnehmen sollten, der Beobachtungszeitraum war 12 Wochen, die Einnahme sollte täglich erfolgen. Nach 12 Wochen wurde die 2. Kontrolluntersuchung durchgeführt, wobei die Patientinnen Auffälligkeiten und Nebenwirkungen zu berichten hatten sowie die Frage beantworten mussten, ob sich die mastalgischen Beschwerden nicht, geringfügig, weitgehend oder vollkommen gebessert hätten.

Diese Beurteilung korreliert mit anderen Publikationen und ist klinisch akzeptiert (Blommers et al., Am. J. Obstet. Gynecol. 187(5) (2002):1389-1394).

### Ergebnisse

Von den 58 Patientinnen kamen 56 nach drei Wochen, um über den Therapieerfolg zu berichten. Nebenwirkungen und Unverträglichkeitserscheinungen wurden nicht registriert, die Patientinnen wiesen auch keine Änderungen des Zyklusverhaltens verglichen zum Zeitraum vor Einnahme des Pflanzenpräparates auf.

Zwei Patientinnen erschienen nicht zur Kontrolluntersuchung und waren auch telefonisch bzw. postalisch nicht kontaktierbar. Von den verbleibenden 56 Patientinnen berichteten 4 über keinen Erfolg. 8 Frauen gaben an, dass sich die mastalgischen Beschwerden geringfügig verändert hätten, bei 24 Frauen konnte eine weitgehende Verbesserung der Mastaglie konstatiert werden, 20 Frauen berichteten, dass die schmerzhaften Brustzustände völlig beseitigt worden waren. Mehr als die Hälfte der Frauen, nämlich 30 Patientinnen, wollten die Behandlung auf eigenen Wunsch weiter fortführen.

Bei 12 Frauen dieser Gruppe wurde nach Beendigung der Chrysin-Behandlung eine Kontrollmammographie durchgeführt. Diese wurde mit einer ein bis zwei Jahren zurückliegenden Mammographie verglichen, bei 6 Frauen konnte der Density Score nach Brisson et al. (Am. J. Epidemol. 115(3) (1982):428-437) angewandt werden, der bei jeder der 6 Patientinnen eine deutliche Verringerung der Brustdichte anzeigte.

Die vorliegende Studie zeigt, dass Chrysin-Präparate effizient zur Behandlung der Mastalgie eingesetzt werden können. Bei der vorliegenden Erfindung werden demgemäß Patientinnen, die an Mastalgie leiden, oder ein Risiko haben dieses zu entwickeln, mit einer effektiven Menge an Chrysin behandelt.

## Patentansprüche

1. Verwendung von Chrysin-Präparaten zur Herstellung eines Medikaments zur Behandlung oder Verhinderung von Mastalgie.

2. Verwendung nach Anspruch 1, **dadurch gekennzeichnet, dass** die Chrysin-Präparate 1 mg bis 10 g, vorzugsweise 5 mg bis 5g, insbesondere 10 mg bis 1 g, Chrysin enthalten.

3. Verwendung nach Anspruch 1 oder 2, **dadurch gekennzeichnet, dass** das Chrysin-Präparat ein oral-einnehmbares Chrysin-Präparat ist, insbesondere in einer Magensaft-resistenten Formulierung.

4. Verwendung nach einem der Ansprüche 1 bis 3, **dadurch gekennzeichnet, dass** die Chrysin-Präparate weiters Phytohormone, vorzugsweise Isoflavone, insbesondere Formononetin, Daidzein oder Genistein, und/oder Gallensäure, insbesondere Cholsäure, enthalten.

5. Verwendung nach einem der Ansprüche 1 bis 4, **dadurch gekennzeichnet, dass** die Chrysin-Präparate Cholsäure in einer Menge von 1 mg bis 10 g, vorzugsweise von 10 mg bis 5 g, insbesondere 100 mg bis 1g, enthalten.

6. Verwendung von Chrysin-Präparaten zur Herstellung eines Nahrungsergänzungsmittels zur Unterstützung der Verhinderung oder Behandlung von Mastalgie.

7. Lebensmittel, enthaltend zugesetztes Chrysin.

8. Lebensmittel nach Anspruch 7, **dadurch gekennzeichnet, dass** es weiters zugesetzte Cholsäure enthält.

9. Verwendung eines Lebensmittels nach Anspruch 7 oder 8 zur Beschleunigung des Fettabbaus.
